# EUROPEAN PATENT APPLICATION

(11) **EP 4 012 048 A1**
(43) Date of publication of application: **15.06.2022**
(21) Application number: 20306533.9
(22) Date of filing: 10.12.2020
(51) Int. Cl.: C12Q 1/6886

(54) **BIOMARKERS FOR PROGNOSING RESPONSE TO TREATMENT AGAINST PANCREATIC DUCTAL ADENOCARNICOMA**

(71) Applicant: INSTITUT NATIONAL DE LA SANTE ET DE LA RECHERCHE MEDICALE (INSERM), 75013 Paris (FR); Centre national de la recherche scientifique, 75016 Paris (FR); Université d'Aix-Marseille, 13007 Marseille (FR); Institut Jean Paoli & Irène Calmettes, 13009 Marseille (FR)
(72) Inventor: IOVANNA, Juan, 13008 MARSEILLE (FR); DUSETTI, Nelson, 13008 MARSEILLE (FR)
(74) Representative: Lavoix

(57) **Abstract**

The present invention relates to a method for determining whether a subject suffering from PDAC will respond favorably to a treatment, in particular to a treatment comprising administering to said subject Gemcitabine, Irinotecan, Oxaliplatin or their combinations, wherein said method comprises assessing the methylation level of a set of CpG sites. Specific CpG signatures have indeed found to be associated to the response to specific treatments. The present invention also relates to a kit suitable for selecting a suitable treatment for a subject suffering from PDAC, as well as the use thereof for determining the methylation status of CpG sites in a sample.

SEQUENCE LISTING

<110>  Université d'Aix-Marseille
       Institut Jean Paoli & Irene Calmettes
       Centre National de la Recherche Scientifique
       INSTITUT NATIONAL DE LA SANTE ET DE LA RECHERCHE MEDICALE
       (INSERM)

<120>  BIOMARKERS FOR PROGNOSING RESPONSE TO TREATMENT AGAINST
       PANCREATIC DUCTAL ADENOCARNICOMA

<130>  BET20P3430

<160>  20

<170>  PatentIn version 3.5

<210>  1
<211>  122
<212>  DNA
<213>  Homo sapiens

<400>  1
ggctcggcgt tgacgtggga cgcggcggag gcgcagcagc cggtggtgat ttgctaacct      60

cgcagcagag aggagttgag ggcgatgaga gcgggtactg cgaactgccg ggcgatgctg     120

tc                                                                    122


<210>  2
<211>  122
<212>  DNA
<213>  Homo sapiens

<400>  2
tgggctccac cgagttccgc ttgcgtcagg cgccttcgcc cctatagcgg ggcggccagc      60

cgcgcacggg cgagttcatc tccaagtcac tttttgtaaa cgccccgcac agcctggacc     120

gg                                                                    122


<210>  3
<211>  122
<212>  DNA
<213>  Homo sapiens

<400>  3
gattcgtatg ggctccaccg agttccgctt gcgtcaggcg ccttcgcccc tatagcgggg      60

cggccagccg cgcacgggcg agttcatctc caagtcactt tttgtaaacg ccccgcacag     120

cc                                                                    122


<210>  4
<211>  122
<212>  DNA
<213>  Homo sapiens

<400>  4
ggctccaccg agttccgctt gcgtcaggcg ccttcgcccc tatagcgggg cggccagccg      60

cgcacgggcg agttcatctc caagtcactt tttgtaaacg ccccgcacag cctggaccgg 120

cc 122

<210> 5
<211> 122
<212> DNA
<213> Homo sapiens

<400> 5
cgccccacac gccggccttt tctgccgctt cccaagccgg agccaatcag cgctcagcag 60

cgaccggcag ggacgaccaa tcgacttatc ctgcgttgtg ctaaagacct agaaatcgag 120

cg 122

<210> 6
<211> 122
<212> DNA
<213> Homo sapiens

<400> 6
gtgctctaac ttaccaaaca cgtggcagcc ggcagaacga aaaggaaaat aaatgaactg 60

cgacgaaata aagcaggcct ttccgcagtc ggccacgtcc aacagccact tggttcctag 120

cg 122

<210> 7
<211> 122
<212> DNA
<213> Homo sapiens

<400> 7
cacccgcagc tgccgacgac cctgccagtc acgccgccgc cggctgcctt tttttagcca 60

cgacatctga ccctggcttc aacagcaccg ttctgggaga gaggtcccag ggcgcgcccg 120

cg 122

<210> 8
<211> 122
<212> DNA
<213> Homo sapiens

<400> 8
gagttctctg gtcttctggc aggggctgag acggcagtcc agcaagggtc acaggcctga 60

cggtatgacc tcttcccctt cccaccgcag gtgcggttct cccggccgga gtcagagcag 120

gc 122

<210> 9
<211> 122
<212> DNA
<213> Homo sapiens

<400>  9
ccaggaacta agggaactct acgctcctac agtcccttta cttactggga tggtgttcac      60

cgcctctgcc cactgagcct gccaaaacaa tctgtcacag ccaagcccaa tcactcccca     120

tt                                                                    122


<210>  10
<211>  122
<212>  DNA
<213>  Homo sapiens

<400>  10
ggagaagtaa gcctctttct tggtctgcta cagacattta tgctaatggt gcttctcttc      60

cgggcctagc tttcacctcc tctgacaggt aatgaactcc ccaagtccaa ttacaggcca     120

cc                                                                    122


<210>  11
<211>  122
<212>  DNA
<213>  Homo sapiens

<400>  11
gctcagaccc cagggcaggc acaggcggtg ggcaggaggt tctgtagcta gtgctgcagt      60

cggcagaaca atgctcccaa gatgtcccct ttctaatccc cggaacctgt gcatatgctt     120

tc                                                                    122


<210>  12
<211>  122
<212>  DNA
<213>  Homo sapiens

<400>  12
cagggcggag ccacagggcc ttgcccgctg tcacacacca agctgggggc agagccctta      60

cgagtgtgga cccagggcag gatgtctcag aacaacaccc tgccagaaac agacacacct     120

gg                                                                    122


<210>  13
<211>  122
<212>  DNA
<213>  Homo sapiens

<400>  13
gactgatgcc cttctcactg cagatccagg ctcaccgagc aggttcctat tcctgatgga      60

cgtggagatg tctgattgga ttttgtcatt tgatttttga cactgattaa tgatagattt     120

tt                                                                    122


<210>  14
<211>  122
<212>  DNA

<213>  Homo sapiens

<400>  14
agggggtgag aaggttggtt ggctggatgg ctgcttcaag taatgcgaaa agtaaaccca      60

cgaaaccacc ttttgaaaac aaagtgcgag ggctgtgcca tcctggctgc agcagggtcg      120

ta                                                                    122


<210>  15
<211>  122
<212>  DNA
<213>  Homo sapiens

<400>  15
atgtcaccca tggagagata aggctctgct agatagaaaa cttgctcatg ggtacctgtc      60

cgtgaggggc ttggcaggtt catggcttta actgtgtgat ttcatgtcgt gtcattcctt      120

tg                                                                    122


<210>  16
<211>  122
<212>  DNA
<213>  Homo sapiens

<400>  16
gggacaatgg tgggaccctc tgcaaactgc ccccagacag actcaatgct taaagcacag      60

cgcagaggct cacagggcct gtcttgtcat gtatgaacac cagggacctt cagagaaggg      120

ct                                                                    122


<210>  17
<211>  122
<212>  DNA
<213>  Homo sapiens

<400>  17
cggggctgtc ttcccgcgga gcgtgctggg ggcgggtcgc gccgggccgg gccgctagtg      60

cgcatgggcg ggcgtcctcg gctctaactg ccgccacttt ccacacgctg ggagggccgt      120

ta                                                                    122


<210>  18
<211>  122
<212>  DNA
<213>  Homo sapiens

<400>  18
tttccctgca gtaactggag tcctcctcgt cgtcttccag ctcctccggg taagggtaat      60

cgaagtcatc gtcgtcctcc tccatctcat cctcctagtt gtcctcagac tcctgggtct      120

cc                                                                    122


<210>  19

<211>  122
<212>  DNA
<213>  Homo sapiens

<400>  19
ccgcccccag acttgcgcat gctgggattg taatactgtc gccttgccac caacgggcta      60

cgagcggtta agagactaca gctcccagga tgtacggtga tggcacccgt ttggactccg     120

cc                                                                    122


<210>  20
<211>  122
<212>  DNA
<213>  Homo sapiens

<400>  20
cgtgaaggcc ggcttccagg gccgccgcag ctactctgag ctggcggagg gcgcggccta      60

cgcgggcgcc tccccgccct ggctgcaggc cgaagcggcc actctcccca agccccgggc     120

ct                                                                    122

## Description

### Field of the invention

The present invention concerns biomarkers for prognosing response to a treatment against pancreatic ductal adenocarcinoma.

### Background of the Invention

Pancreatic ductal adenocarcinoma (PDAC) is the fourth leading cause of cancer-related mortality in western countries and bears one of the poorest prognoses with a five-year survival rate of 6% In addition to frequent late-stage diagnosis, this dismal outcome can also be explained by the lack of effective therapies. Targeted therapies and immunotherapies have failed to improve unselected patients' outcomes, making chemotherapy the only effective systemic treatment. Despite being a generally refractory cancer, it has been shown that the molecularly guided selection of small patient subgroups increased the efficacy for some therapies such as olaparib for patients with germline BRCA mutations (approximately 5% of patients) or immunotherapy for mismatch-repair deficient tumors (approximately 1% of patients). These studies demonstrate the advantage of molecular stratification for therapeutic decisions and introduce an incremental model for PDAC, solving one subgroup at a time.

For patients with excellent performance status, the polychemotherapy regimen FOLFIRINOX was shown to be more effective than gemcitabine alone in both the adjuvant and metastatic settings at the expense of high toxicities. The selection of systemic therapy is therefore based on patient's fitness rather than on the potential efficacy of a particular chemotherapeutic regimen. Companion diagnostics are used to guide the choice of therapy by selecting patients that have a higher chance of responding to a given therapeutic agent. With an ever-growing number of regimens composed of multiple chemotherapies, this has not only the potential to improve survival by matching drugs to likely responders, but also to reduce adverse effects by avoiding unnecessary highly toxic regimens.

Different chemotherapy drugs are currently used for treating pancreatic cancer.

Gemcitabine is the preferred companion of nab-paclitaxel, another systemic therapy used as the first or second line in the advanced setting. The AFUGEM study suggested that 5-FU could be equivalent to gemcitabine in association with nab-paclitaxel highlighting the need of gemcitabine/5-FU predictive biomarkers. Furthermore, there is to date no upfront comparison of modified FOLFIRINOX and gemcitabine-nabpaclitaxel. It could be proposed that patients with homologous recombination deficiency are to be treated with platinum salt while for the others a gemcitabine efficacy predictive biomarker could help select the patients for the gemcitabine-nabpaclitaxel regimen.

In addition, gemcitabine remains to date the most effective monotherapy in PDAC with an estimated response rate of 10% to 23% in advanced patients and is often used in patients unfit for more aggressive therapies. Mostly based on gemcitabine metabolism, the association of single gene biomarkers with the response to gemcitabine has been demonstrated both at the level of protein expression and genetic polymorphisms. In particular, the nucleoside transporter hENT1 has been extensively studied and its protein expression was shown in multiple studies to be associated with gemcitabine sensitivity in PDAC as well as in other malignancies. The stratification of patients by hENT1 expression, however, is hindered by the difficulty to robustly assess the protein's expression given the high discrepancies between available antibodies.

Multi-gene signatures based on RNA expression measurements have been shown to provide robust predictive tools in breast and prostate cancer. In PDAC, RNA signatures provide an in-depth description of tumor phenotypes with robust prognostic and suggested predictive value.

FOLFIRINOX is one of the main chemotherapy treatments for pancreatic ductal adenocarcinoma. FOLFIRINOX is the combination of three different chemotherapy drugs and a vitamin: fluorouracil (5-FU), irinotecan, and oxaliplatin. It also includes folinic acid (leucovorin). This is a vitamin that helps fluorouracil work well.

FOLFIRINOX regime produce strong side effects such as: increased risk of an infection due to a drop in white blood cells, tiredness and breathlessness due to a drop in red blood cells, bruising more easily due to a drop in platelets also you may have bleeding gums, nosebleeds or tiny red spots known as petechial, tiredness during treatment and sometimes for months afterwards, feeling or being sick (you will be given anti-sickness tablets to help with this), diarrhea, hair loss or hair thinning, sore mouth and ulcers, some liver changes (mild and unlikely to cause symptoms), gritty or watery eyes, blurred vision, sore, red peeling on palms of hands and soles of the feet (known as palmar-plantar syndrome), a brown skin marking following the line of the vein where chemotherapy was injected, skin rashes which may cause itching and some women may find their periods stop (amenorrhoea) this is often temporary, as the most frequents.

Only one third of patients treated with FOLFIRINOX respond to the treatment. The remaining two third suffer of the side effects of FOLFIRINOX without any benefice. This is why to select FOLFIRINOX-responders patients is a main challenge.

Epigenetic alteration of gene expression can be achieved by DNA methylation, which is known to be deregulated in PDAC resulting in altered gene expression, genome structure reorganization, tumor grade, tumor stage and survival time of patients. The Cancer Genome Atlas (TCGA) and International Cancer Genome Consortium (ICGC) have generated methylome data for thousands of tumor samples spanning across -25 cancer types including several PDAC. More recently a DNA Methylation dataset on more than 750,000 CpG was generated in our laboratory with genomic DNA obtained from pure human PDAC cells as well as patients derived tumor xenograft (PDX). This is an invaluable material since it will let to analyze DNA Methylation specifically on tumor DNA.

Earlier studies on PDAC methylation analysis have used limited patient samples or CpG sites, for example for assessing a CpG methylation profile in pancreatic intraductal neoplasia or pancreatic cancer genome or a correlation between DNA methylation and survival of PDAC patients.

There is therefore a need to provide effective tools to predict gemcitabine and FOLFIRINOX sensitivity in a patient suffering from PDAC, in order to select the most suitable treatment.

### Description

The Inventors have surprisingly found that DNA methylation landscape of PDAC tumors is associated to the sensitivity to irinotecan and oxaliplatin, two chemotherapy drugs present in the FOLFIRINOX regime, as well as to gemcitabine. Several models of PDAC primary cells and PDX were indeed treated with the anticancer drugs (gemcitabine, irinotecan and oxaliplatin) and their sensitivity was correlated with their methyloma. The Inventors found several differentially methylated CpGs able to predict the sensitivity to gemcitabine, irinotecan and oxaliplatin, which were then validated on PDX treated with these drugs.

The Inventors have thus determined sets of CpG sites able to predict the gemcitabine and FOLFIRINOX sensitivity in PDAC. These set of CpG sites can thus be used for selecting the most suitable treatment to a given patient suffering from PDAC.

A first object of the present invention thus relates to a method for determining whether a subject suffering from PDAC will respond favorably to at least one treatment, comprising:
(i) providing at least one set of CpG sites whose methylation level is associated to the response to said treatment,
(ii) determining the methylation level of the CpG sites of said set in a sample from said subject, preferably in a DNA sample from said subject, and
(iii) deducing from the results of step (ii) whether the subject will respond favorably to said treatment.

Said treatment may comprise Gemcitabine, Irinotecan, Oxaliplatin, Fluorouracil (5-FU), Cisplatin, Abraxane and/or Taxol.

The sample preferably originates from the pancreatic ductal adenocarcinoma of said subject.

In the method as defined above, step (i) preferably comprises providing a set of CpG sites whose methylation level is associated to the response to Gemcitabine, a set of CpG sites whose methylation level is associated to the response to Irinotecan and/or a set of CpG sites whose methylation level is associated to the response to Oxaliplatin.

The set of CpG sites whose methylation level is associated to the response to Gemcitabine may for example comprises at least four CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7.

The set of CpG sites whose methylation level is associated to the response to Irinotecan may for example comprise at least four CpG sites selected from the group consisting of the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16.

The set of CpG sites whose methylation level is associated to the response to Oxaliplatin may for example comprise at least three CpG sites selected from the group consisting of the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20.

Another object of the present invention is a kit suitable for determining whether a subject suffering from PDAC will respond favorably to a treatment, wherein said kit comprises:
(i) means for assessing the methylation level of a set of at most 60 CpG sites, wherein the CpG sites comprise:
   - at least four CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7, and/or
   - at least four CpG sites selected from the group consisting the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16, and/or
   - at least three CpG sites selected from the group consisting the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20, and
(ii) optionally, means for extracting DNA from a sample.

The means for assessing the methylation level of the CpG sites may for example comprise:
- bisulfite,
- means for DNA amplification, and/or
- probes able to detect a methylated cytosine and/or an unmethylated cytosine at each CpG site of the set of CpG sites.

Another object of the present invention is the use of the kit as defined above for determining the methylation level of CpG sites in a sample, in particular for determining whether a subject suffering from PDAC will respond favorably to a treatment.

Another object of the present invention is a pharmaceutical composition for use in the treatment of PDAC in a subject, wherein said pharmaceutical composition comprises a treatment suitable for said subject, wherein said suitable treatment is selected by performing the method for determining whether a subject suffering from PDAC will respond favorably to at least one treatment, said method being as defined above, and selecting a treatment to which the subject will respond favorably.

### Subject

The subject is a subject suffering from PDAC (*Pancreatic ductal adenocarcinoma*).

The subject is preferably a human subject, also referred to as "patient".

The subject may be of any age and gender.

The subject may have being diagnosed as suffering from PDAC by any method well known by the skilled person, such as imaging technique(s) (for example contrast-enhanced computed tomography (CT) scan of the abdominal cavity and pelvis, magnetic resonance imaging (MRI) and/or Endoscopic ultrasound (EUS)) and/or an histological analysis of a biopsy of the pancreatic lesions.

PDAC is for example characterized by a particular short time of survival and therefore given not multiple therapeutic options.

The subject is thus a subject in need of a treatment for treating PDAC.

### Biological sample

The method for determining whether a subject suffering from PDAC will respond favorably to at least one treatment is carried out on a sample from the subject, the subject being as defined above.

The sample is thus a biological sample.

The sample preferably originates from the pancreatic ductal adenocarcinoma of said subject.

The sample may be a biopsy sample or a sample from the pancreatic ductal adenocarcinoma, which was for example removed by surgery.

### Determining the methylation level of CpG sites

The present invention is based on determining the methylation level of a set of CpG sites.

Any suitable method well-known by the skilled person may be used for determining the methylation level of a CpG site.

For example, determining the methylation level of a CpG site may be carried out by (i) bisulfite treatment followed by sequencing, detection by a probe, for example on an array, or pyrosequencing, or by (ii) liquid chromatography coupled with tandem mass spectrometry.

Treatment of DNA with bisulfite converts unmethylated cytosine residues to uracil, but leaves 5-methylcytosine residues unaffected.

In one preferred embodiment, determining the methylation level of a set of CpG sites may comprise:
- optionally, extracting DNA from a sample, in particular from a sample as defined above, to obtain a DNA sample,
- adding bisulfite, in particular sodium bisulfite, to the DNA sample, in particular to convert unmethylated cytosine residues to uracil, to obtain a treated DNA sample,
- amplifying DNA of the treated DNA sample, for example by PCR, in particular amplifying the DNA comprising the CpG sites of said set, to obtain an amplified DNA sample, and
- determining the level of methylation of each CpG site of said set in the amplified DNA sample.

Amplifying DNA is preferably performed using primers able to amplify each CpG site of said set of CpG sites. The primers used to amplify a given CpG site may hybridize to DNA regions flanking said CpG site. Alternatively, one of the primers used to amplify a given CpG site may hybridize to a DNA region comprising the CpG site in its methylated stated (in particular comprising a cytosine) and/or one of the primers used to amplify a given CpG site may hybridize to a DNA region comprising the CpG site in its unmethylated (in particular comprising an uracil instead of a cytosine, further to the bisulfite treatment).

In said amplified DNA sample, the presence of a cytosine at a CpG site indicates a methylated cytosine and the presence of an uracil at a CpG site indicates an unmethylated state.

The level of methylation of a given CpG site may be an hypermethylation or an hypomethylation.

The level of methylation of a given CpG site is an hypermethylation when the number of methylated cytosine at said given CpG site is greater than the number of unmethylated cytosine at said given CpG site, in the DNA sample.

The level of methylation of a given CpG site is an hypomethylation when the number of unmethylated cytosine at said given CpG site is greater than the number of methylated cytosine at said given CpG site, in the DNA sample.

### Method for determining whether a subject suffering from PDAC will respond favorably to at least one treatment

The present invention thus relates to a method for determining whether a subject suffering from PDAC will respond favorably to at least one treatment.

By "a method for determining whether a subject suffering from PDAC will respond favorably to at least one treatment", it is herein particularly meant a method for determining a suitable treatment to a subject, i.e. a treatment active against PDAC in said subject.

The present invention particularly relates to a method for determining whether a subject suffering from PDAC will respond favorably to at least one treatment, comprising:
(i) providing at least one set of CpG sites whose methylation level is associated to the response to said treatment,
(ii) determining the methylation level of the CpG sites of said set in a sample from said subject, and
(iii) deducing from the results of step (ii) whether the subject will respond favorably to said treatment.

The method may for example determine whether a subject suffering from PDAC will respond favorably to one treatment or to at least two different treatments, in particular to allow selecting the most suitable treatment among the possible treatments.

The treatment may be any treatment available for the prevention and/or treatment of PDAC.

The treatment may for example comprise Gemcitabine or a derivative thereof, Irinotecan or a derivative thereof, Oxaliplatin or a derivative thereof, Fluorouracil (5-FU) or a derivative thereof, Cisplatin or a derivative thereof and/or Taxol, for example in the form Abraxane, or a derivative thereof.

The present invention preferably relates to a method for determining whether a subject suffering from PDAC will respond favorably to (i) Gemcitabine or a derivative thereof, (ii) Irinotecan or a derivative thereof and/or (iii) Oxaliplatin or a derivative thereof.

Gemcitabine is the compound 2'-deoxy-2',2'-difluoro-cytidine, also referred to as CAS number 95058-81-4.

A derivative of gemcitabine is a compound obtained for example by at least one chemical modification from gemcitabine. The derivative of gemcitabine is preferably at least as efficient as gemcitabine and/or has less side effects.

Irinotecan is the compound [1,4'-Bipiperidine]-1'-carboxylic acid, also referred to as CPT-11 or under CAS Number 100286-90-6. Irinotecan is preferably used in the form of irinotecan hydrochloride trihydrate.

A derivative of Irinotecan is a compound obtained for example by at least one chemical modification from Irinotecan. The derivative of Irinotecan is preferably at least as efficient as Irinotecan and/or has less side effects.

Oxaliplatin is the compound [SP-4-2-(1R-trans)]-(1,2-Cyclohexanediamine-N,N')[ethanedioata(2--)-O,O']platinum, also referred to as CAS number 61825-94-3.

A derivative of Oxaliplatin is a compound obtained for example by at least one chemical modification from Oxaliplatin. The derivative of Oxaliplatin is preferably at least as efficient as Oxaliplatin and/or has less side effects.

Fluorouracil is the compound 5-Fluoropyrimidine-2,4-diol, also referred to as (5-FU) or under CAS number 51-21-8.

A derivative of Fluorouracil is a compound obtained for example by at least one chemical modification from Fluorouracil. The derivative of Fluorouracil is preferably at least as efficient as Fluorouracil and/or has less side effects

Cisplatin is the compound cis-diamminedichloroplatinum (II), also referred to under CAS number 15663-27-1.

A derivative of Cisplatin is a compound obtained for example by at least one chemical modification from Cisplatin. The derivative of Cisplatin is preferably at least as efficient as Cisplatin and/or has less side effects.

Taxol, also called Paclitaxel, is a compound referred to as CAS number 33069-62-4.

Taxol is preferably used in the form of Albumin-bound paclitaxel, also called Abraxane or nab-paclitaxel.

A derivative of Taxol is a compound obtained for example by at least one chemical modification from Taxol. The derivative of Taxol is preferably at least as efficient as Taxol and/or has less side effects. An example of Taxol derivative is for example placlitaxel.

### Step (i)

Step (i) comprises providing at least one set of CpG sites whose methylation level is associated to the response to said treatment.

These CpG sites are thus biomarkers useful for prognosing response to a treatment against pancreatic ductal adenocarcinoma.

The methylation level of the CpG sites of a set associated to a favorable response to treatment is also referred to as the CpG signature associated to a favorable response to treatment.

The set of CpG site may for example be a set of CpG sites whose methylation level is associated to the response to Gemcitabine, a set of CpG sites whose methylation level is associated to the response to Irinotecan or a set of CpG sites whose methylation level is associated to the response to Oxaliplatin.

Step (i) may for example comprises providing one set of CpG sites whose methylation level is associated to the response to Gemcitabine, one set of CpG sites whose methylation level is associated to the response to Irinotecan, one set of CpG sites whose methylation level is associated to the response to Oxaliplatin and/or at least one set of CpG sites whose methylation level is associated to the response to another treatment.

The set of CpG sites whose methylation level is associated to the response to Gemcitabine preferably comprises at least four CpG sites, more preferably at least five CpG sites, still more preferably at least six CpG sites, selected from the group consisting of: the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7.

In a preferred embodiment, the set of CpG sites whose methylation level is associated to the response to Gemcitabine comprises or consists of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7.

The set of CpG sites whose methylation level is associated to the response to Irinotecan preferably comprises at least four CpG sites, more preferably at least five, at least six or at least seven CpG sites, still more preferably at least eight CpG sites, selected from the group consisting of: the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16.

In a preferred embodiment, the set of CpG sites whose methylation level is associated to the response to Irinotecan comprises or consists of the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16

The set of CpG sites whose methylation level is associated to the response to Oxaliplatin preferably comprises at least three CpG sites selected from the group consisting of the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20.

In a preferred embodiment, the set of CpG sites whose methylation level is associated to the response to Oxaliplatin comprises or consists of the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20.

### Step (ii)

Step (ii) comprises determining the methylation level of the CpG sites of said set in a sample from said subject.

The sample is particularly as defined above.

The sample is preferably a DNA sample.

The sample preferably originates from the pancreatic ductal adenocarcinoma of said patient.

Determining the methylation level of the CpG sites of said set in a sample from said subject may be performed as disclosed above in the section "*Determining the methylation level of CpG sites".*

### Step (iii)

Step (iii) comprises deducing from the results of step (ii) whether the subject will respond favorably to said treatment.

Step (iii) preferably comprises, for each set of CpG sites provided in step (i):
- comparing the level of methylation of the CpG sites determined in step (ii) to the level of methylation of said CpG sites associated to a favorable response to treatment,
- if the level of methylation of the CpG sites determined in step (ii) is the same as the level of methylation of said CpG sites associated to a favorable response to treatment, deducing from the results of step (ii) that the subject will respond favorably to the treatment (i.e. the treatment associated to the level of methylation of said set of CpG sites) and if the level of methylation of the CpG sites determined in step (ii) is different from the level of methylation of said CpG sites associated to a favorable response to treatment, deducing from the results of step (ii) that the subject will not respond favorably to the treatment (i.e. the treatment associated to the level of methylation of said set of CpG sites).

Tables 1, 2 and 3 below indicate the CpG signature associated to a favorable response to Gemcitabine, Irinotecan and Oxaliplatin, respectively.

**Table 1: CpG signature associated to a favorable response to Gemcitabine**

| **N° sequence** | **Marker** | **Sensitive** | **Position of the CpG** |
|---|---|---|---|
| SEQ ID NO: 1 | cg09719477 | Hypermethylation | 61-62 |
| SEQ ID NO: 2 | cg04111064 | Hypermethylation | 61-62 |
| SEQ ID NO: 3 | cg15720669 | Hypermethylation | 61-62 |
| SEQ ID NO: 4 | cg17802942 | Hypermethylation | 61-62 |
| SEQ ID NO: 5 | cg10190444 | Hypermethylation | 61-62 |
| SEQ ID NO: 6 | cg05471523 | Hypermethylation | 61-62 |
| SEQ ID NO: 7 | cg19635869 | Hypermethylation | 61-62 |

When step (i) comprises providing a set of CpG sites whose methylation level is associated to the response to Gemcitabine, step (iii) comprises deducing from the results of step (ii) that:
- the subject will respond favorably to Gemcitabine if the level of methylation is hypermethylation for each of the CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7 and
- the subject will not respond favorably to the Gemcitabine if the level of methylation is hypomethylation for at least one of the CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7.

**Table 2: CpG signature associated to a favorable response to Irinotecan**

| **N° sequence** | **Marker** | **Sensitive** | **Position of the CpG** |
|---|---|---|---|
| SEQ ID NO: 8 | cg26807301 | Hypomethylation | 61-62 |
| SEQ ID NO: 9 | cg02940693 | Hypomethylation | 61-62 |
| SEQ ID NO: 10 | cg08803589 | Hypomethylation | 61-62 |
| SEQ ID NO: 11 | cg04442017 | Hypomethylation | 61-62 |
| SEQ ID NO: 12 | cg11277783 | Hypomethylation | 61-62 |
| SEQ ID NO: 13 | cg18270446 | Hypermethylation | 61-62 |
| SEQ ID NO: 14 | cg12974599 | Hypermethylation | 61-62 |
| SEQ ID NO: 15 | cg17474302 | Hypermethylation | 61-62 |
| SEQ ID NO: 16 | cg02306236 | Hypermethylation | 61-62 |

When step (i) comprises providing a set of CpG sites whose methylation level is associated to the response to Irinotecan, step (iii) comprises deducing from the results of step (ii) that:
- the subject will respond favorably to Irinotecan if the level of methylation is hypomethylation for each of the CpG sites selected from the group consisting of the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11 and the CpG located at position 61-62 of sequence SEQ ID NO: 12 and if the level of methylation is hypermethylation for each of the CpG sites selected from the group consisting of the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16, and
- the subject will not respond favorably to the Irinotecan if the level of methylation is hypermethylation for at least one of the CpG sites selected from the group consisting of the CpG located at position 61-62 of SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11 and the CpG located at position 61-62 of sequence SEQ ID NO: 12 or if the level of methylation is hypomethylation for at least one of the CpG sites selected from the group consisting of the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16.

**Table 3: Cpg signature associated to a favorable response to Oxaliplatin**

| **N° sequence** | **Marker** | **Sensitive** | **Position of the CpG** |
|---|---|---|---|
| SEQ ID NO: 17 | cg27205904 | Hypermethylation | 61-62 |
| SEQ ID NO: 18 | cg16023943 | Hypomethylation | 61-62 |
| SEQ ID NO: 19 | cg23849078 | Hypomethylation | 61-62 |
| SEQ ID NO: 20 | cg26981809 | Hypomethylation | 61-62 |

When step (i) comprises providing a set of CpG sites whose methylation level is associated to the response to Oxaliplatin, step (iii) comprises deducing from the results of step (ii) that:
- the subject will respond favorably to Oxaliplatin if the level of methylation is hypermethylation for the CpG located at position 61-62 of sequence SEQ ID NO: 17 and if the level of methylation is hypomethylation for each of the CpG sites selected from the group consisting of the CpG located at position 61-62 of sequence SEQ ID NO: 18, the CpG located at position 61-62 of sequence SEQ ID NO: 19 and the CpG located at position 61-62 of SEQ ID NO: 20, and
- the subject will not respond favorably to the Oxaliplatin if the level of methylation is hypomethylation for the CpG located at position 61-62 of SEQ ID NO: 17 or if the level of methylation is hypermethylation for at least one of the CpG sites selected from the group consisting of the CpG located at position 61-62 of sequence SEQ ID NO: 18, the CpG located at position 61-62 of sequence SEQ ID NO: 19 and the CpG located at position 61-62 of SEQ ID NO: 20.

### Method of treatment of PDAC in a subject

The present invention also relates to a method for the treatment of PDAC in a subject comprising:
- determining whether the subject will respond favorably to at least one treatment, by performing the steps of the method for determining whether a subject will respond favorably to at least one treatment as defined above,
- selecting a treatment to which the subject will respond favorably, and
- administering to said subject said selected treatment.

The subject is particularly as defined above.

The present invention also relates a pharmaceutical composition for use in the treatment of PDAC in a subject, wherein said pharmaceutical composition comprises a treatment suitable for said subject, wherein said suitable treatment is selected by performing the method for determining whether a subject suffering from PDAC will respond favorably to at least one treatment as defined above and selecting a treatment to which the subject will respond favorably.

The treatment may for example comprises Gemcitabine or a derivative thereof, Irinotecan or a derivative thereof, Oxaliplatin or a derivative thereof, Fluorouracil (5-FU) or a derivative thereof, Cisplatin or a derivative thereof and/or Taxol, for example in the form of Abraxane, or a derivative thereof.

If step (iii) comprises deducing that the subject suffering from PDAC will respond favorably to at least one treatment, preferably at least two treatments, selected form the group consisting of fluorouracil (5-FU), irinotecan and oxaliplatin, the selected treatment may be FOLFIRINOX.

### Kit

The present invention also relates to a kit suitable for determining whether a subject suffering from PDAC will respond favorably to at least one treatment.

This kit as defined above preferably comprises:
- means for assessing the methylation level of at least three or at least four CpGs sites of at least one set of CpG sites whose methylation level is associated to the response to a treatment, and
- optionally, means for extracting DNA from a sample.

The kit as defined above preferably comprises means for assessing the methylation level of at most 60 CpG sites, more preferably at most 50 CpGs, still more preferably at most 40 CpG sites or at most 30 CpG sites.

The present invention particularly relates to a kit suitable for determining whether a subject suffering from PDAC will respond favorably to a treatment, wherein said kit comprises:
(i) means for assessing the methylation level of a set of at most 60 CpG sites, wherein the CpG sites comprise:
   - at least four CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7, and/or
   - at least four CpG sites selected from the group consisting of the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16, and/or
   - at least three CpG sites selected from the group consisting of the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20, and
(ii) optionally, means for extracting DNA from a sample.

Means for extracting DNA for example are well known by the skilled person.

The means for assessing the methylation level of the CpG sites may for example comprise:
- bisulfite, in particular sodium bisulfite,
- means for DNA amplification, and/or
- probes able to detect a methylated cytosine and/or an unmethylated cytosine at each CpG site of the set of CpG sites.

The probes for example comprises:
- 7 probes specifically hybridizing to 7 nucleic acids comprising at least a fragment of sequences SEQ ID NO: 1 to 7, respectively, wherein said fragment comprises a cytosine at position 61 and/or 7 probes specifically hybridizing to 7 nucleic acids comprising at least a fragment of sequences SEQ ID NO: 1 to 7, respectively, wherein said fragment comprises an uracil at position 61,
- 9 probes specifically hybridizing to 9 nucleic acids comprising at least a fragment of sequences SEQ ID NO: 8 to 16, respectively, wherein said fragment comprises a cytosine at position 61 and/or 9 probes specifically hybridizing to 9 nucleic acids comprising at least a fragment of sequences SEQ ID NO: 8 to 16, respectively, wherein said fragment comprises a uracil at position 61, and/or
- 4 probes specifically hybridizing to 4 nucleic acids comprising at least a fragment of sequences SEQ ID NO: 17 to 20, respectively, wherein said fragment comprises a cytosine at position 61 and/or 4 probes specifically hybridizing to 4 nucleic acids comprising at least a fragment of sequences SEQ ID NO: 17 to 20, respectively, wherein said fragment comprises an uracil at position 61.

Means for DNA amplification for example comprise primers, a DNA polymerase, dNTPs and/or a buffer solution.

The primers used for DNA amplification are preferably able to amplify each CpG site of said set of CpG sites. The primers used to amplify a given CpG site may hybridize to DNA regions flanking said CpG site. Alternatively, one of the primers used to amplify a given CpG site may hybridize to a DNA region comprising the CpG site in its methylated stated (in particular comprising a cytosine) and/or one of the primers used to amplify a given CpG site may hybridize to a DNA region comprising the CpG site in its unmethylated (in particular comprising an uracil instead of a cytosine, further to the bisulfite treatment).

### Use of a kit as defined above

The present invention also relates to the use of a kit as defined above for determining the methylation level of CpG sites in a sample, in particular for determining whether a subject suffering from PDAC will respond favorably to a treatment.

The present invention thus also relates to the *in vitro* use of a kit as defined above for determining whether a subject suffering from PDAC will respond favorably to a treatment.

### Figures

Figure 1: Strategy
Figure 2: Training Cohort Signature Identification. A. Gemcitabine. B. Irinotecan. C. Oxaliplatin
Figure 3: Signature Validation *in vivo.* A. Gemcitabine. B. Irinotecan. C. Oxaliplatin

### EXAMPLE

### Methods

### Patient's cohort

All patients presenting with a suspicion of PDAC between February 2011 and September 2015 and that were included in the clinical trial "Predictive Biomarkers of Therapeutic Response in Pancreatic Tumors" (NCT01692873) were included in this study after receiving ethics review board approval. In total, 38 patients diagnosed with PDAC were included in this study.

### Production of patient derived tumor xenograft (PDX)

All animal experiments were conducted in accordance with institutional guidelines and were approved by the "Plateforme de Stabulation et d'Experimentation Animale" (PSEA, Scientific Park of Luminy, Marseille). PDAC tissue was fragmented, mixed with 100 µL of Matrigel and implanted with a 10 gauge trocar (Innovative Research of America, Sarasota, FL) in the subcutaneous right upper flank of an anesthetized male NMRI-nude mouse (Swiss Nude Mouse Crl: NU(Ico)-Foxn1nu; Charles River Laboratories, Wilmington, MA). Once xenografts reached 1 cm3, they were removed and passed to NMRI-nude mice.

### DNA isolation method

DNA has been extracted from 27 PDX considered as the training cohort and 11 PDX considered as the validation cohort. Approximately 50 mg fragments of frozen tissues were transferred into homogenization tubes (Precellys lysing kit CK28R, Bertin Technologies) containing 160 µl PBS (Sigma-Aldrich^{™}). Tissues were homogenized with the Precellys 24-Dual homo-genizer (Bertin Technologies) three times 15 s at 6500 rpm speed with 10 s pause between homogenization steps. A digestion step was then performed at 56°C during 2 h in ATL buffer and proteinase K solution. Finally, DNA was purified using the QIAamp DNA Mini kit (QiagenTM) according to the manufacturer's instructions.

### CpG methylation profiling of PDAC PDX

IlluminaInfinium HumanMethylation450 chips were used to measure genome-wide CpG methylation profiles following manufacturer's instructions. Beta-values were extracted using Illumina's GenomeStudio software. Probes containing single-nucleotide and indel polymorphism or overlapping with a repetitive element that was not uniquely aligned to the human genome were removed.

### Xenografted Tumor Processing for obtaining primary cells culture

Twenty eight xenografts obtained from mice were split into several small pieces and used for cell culture. These fragments were processed in a biosafety chamber: after fine mincing, they were treated with collagenase type V (ref C9263; Sigma-Aldrich, St. Louis, MO) and trypsin/EDTA (ref 25200-056; Gibco, Life Technologies, Grand Island, NY) and were suspended in Dulbecco's modified Eagle's medium supplemented with 1% w/w penicillin/streptomycin (Gibco, Life Technologies) and 10% fetal bovine serum (Lonza Inc., Walkersville, MD). After centrifugation, cells were resuspended in serum-free ductal media at 37°C in a 5% CO2 incubator. Amplified cells were stored in liquid nitrogen. Cells were weaned from antibiotics for ≥48 hours before testing.

### Chemograms

PDX-derived cell lines were screened for chemosensitivity to three drugs: gemcitabine (Eli Lilly & Co., Indianapolis, IN), oxaliplatin (Hospira, Lake Forest, IL) and irinotecan active metabolite named 7-ethyl 10-hydroxycamptothecin or SN-38 (11406; Sigma-Aldrich). These cells were treated for 72 hours with increasing concentrations of chemotherapeutic drugs ranging from 0 to 1000 µmol/L. Five thousand cells were plated per well in 96-well plates in serum free defined media. Twenty-four hours later, the media were supplemented with increasing concentrations of drugs and were incubated for an additional 72 hours. Each experiment was performed in triplicate and repeated at least three times.

Cell viability was estimated after addition of the PrestoBlue cell viability reagent (Life Technologies) for 3 hours following the protocol provided by the supplier. Cell viability was measured on days 0 and 3 to calculate the replication rate of the cells. Area under the curve (AUC) was calculated using GR metrics R package.

### in vivo PDX models of chemosensitivity in PDX

Each PDX corresponding to one patient was inoculated into nude NMRI mice for each treatment. A total of 32 animals for each of the 8 models (total = 256 animals) were utilized. When the PDX for each patient reached 200 mm3 animals were randomized and treated with gemcitabine (120 mg/kg every third day for four administrations in the tail vein, n = 8), irinotecan (22 mg/kg every second day for three administrations in the tail vein, n = 8), and oxaliplatin (5 mg/kg every fourth day for two administrations in the tail vein, n = 8) or vehicle (NaCI 0.9% n = 8). Chemotherapy sensitivity was revealed by tumor growth monitoring. Tumor size was measured with a Vernier caliper twice weekly, and the tumor volume was calculated with the equation v = (length/width2)/2. The "Percentage of Resistance" for each PDX and drug was calculated as the ratio among the AUC of treated animals over the AUC of untreated animals.

### Statistical and Independent component analysis

Methylation Beta values of 773,234 CpGs from 27 (training cohort) and 11 (validation cohort) patients were included in the component analysis. Independent component analysis (ICA) was performed using the JADE (joint approximate diagonalization of eigenmatrices) algorithm. We used different ICA systems with an increasing number of components from k=2 to k=20. The component selection for each reported drug was performed, applying Spearman's correlation between the individual contribution of each PDX on the specific component and the AUC of the PDX-derived cell line. The component projection was made using the cross-product among the Moore-Penrose generalized inverse of the gene contribution and the metabolome matrix.

### Results

### Generation of methylome signatures for Gemcitabine, Irinotecan, and Oxaliplatin

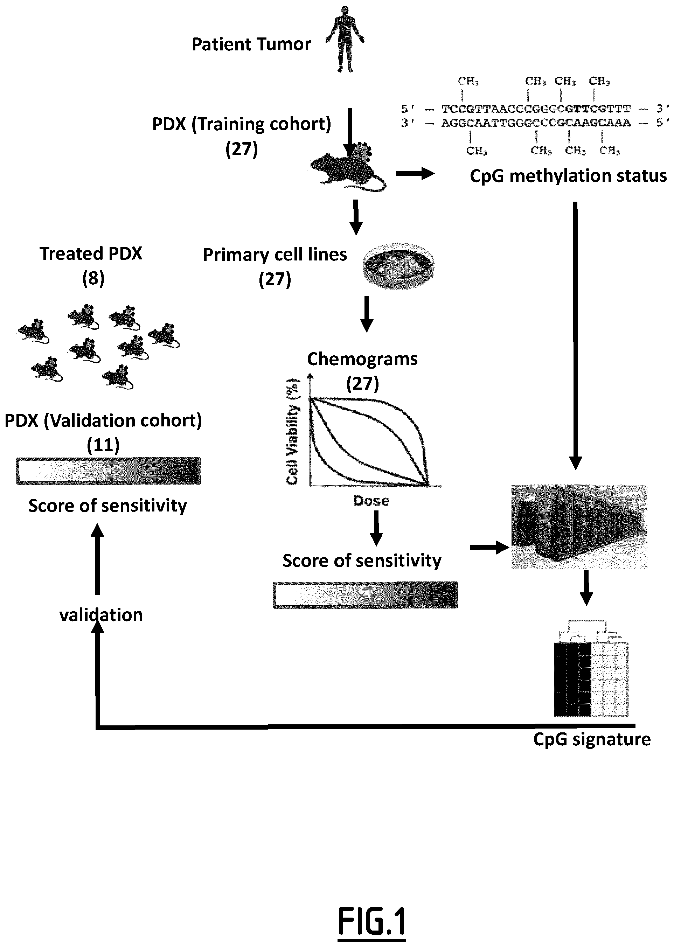

PDAC methylomic signatures were generated for Gemcitabine, Irinotecan, and Oxaliplatin on 38 PDX. These PDX were split into 27 and 11, representing the training cohort and validation cohort, respectively (*see* *Figure 1* *for the employed strategy*)*.*

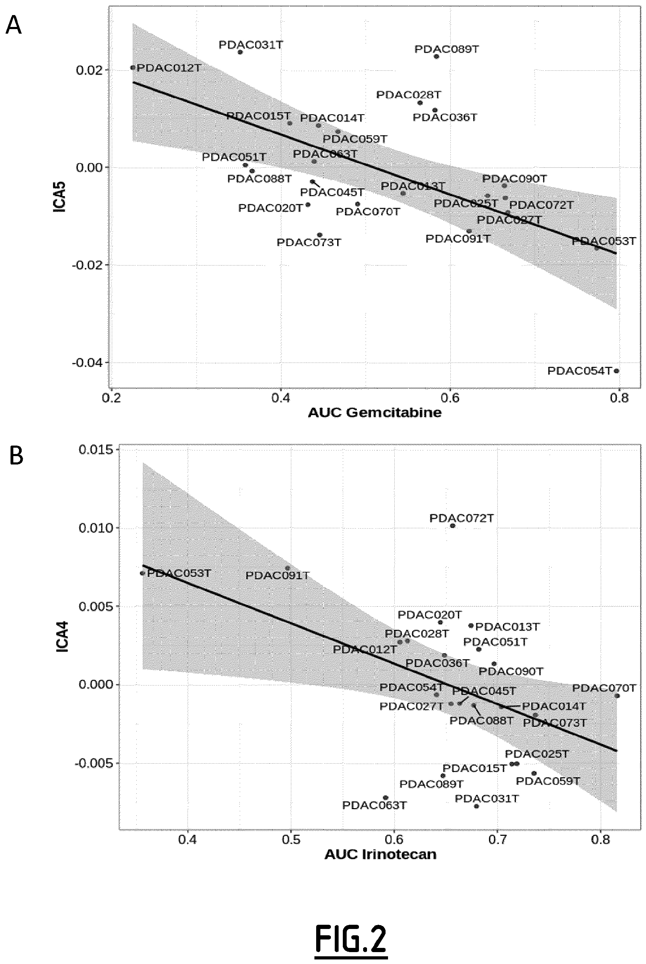

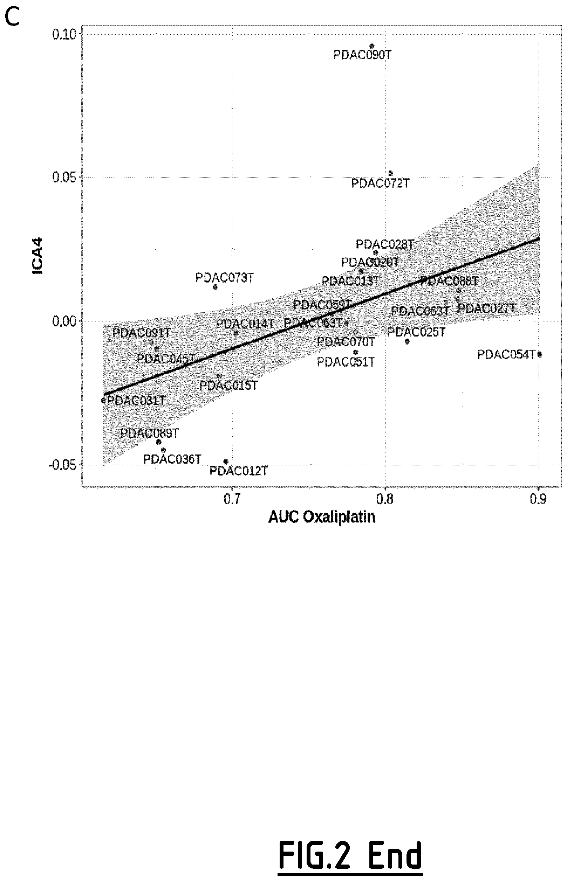

Primary cell lines were derived from the training cohort to perform the chemograms and characterize the PDX chemosensitivity profiles. The reported signatures were extracted applying ICA analysis on the training cohort methylome, followed by Spearman's correlation between the PDX methylome contribution and the AUC calculated from the PDX-derived cell lines. Gemcitabine (r = -0.51; P = 0.01; Figure 2A), Irinotecan (r = -0.46; P = 0.03; Figure 2B), and Oxaliplatin (r = 0.54; P = 0.01; Figure 2C) signatures showed a strong predictive power, regarding to the chemosensitivity measured trough the cell lines. The most contributive CpGs corresponded to 263 for Gemcitabine, 149 for Irinotecan and 95 for Oxaliplatin (*data not shown*).

After that, the signatures were refined, isolating the CpGs with the higher predictive power assessed for the global component's contribution and their distribution between resistant and sensitive tumors (*see Table 1*).

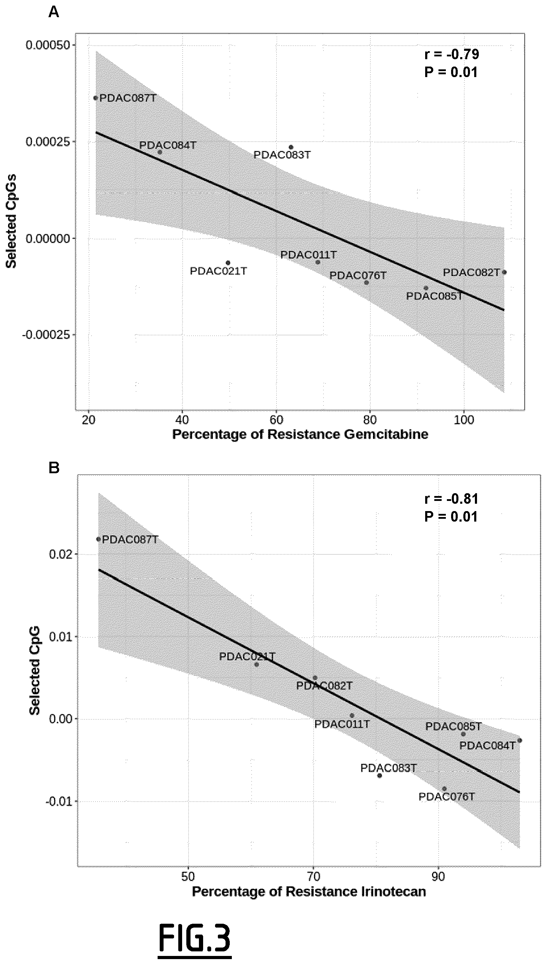

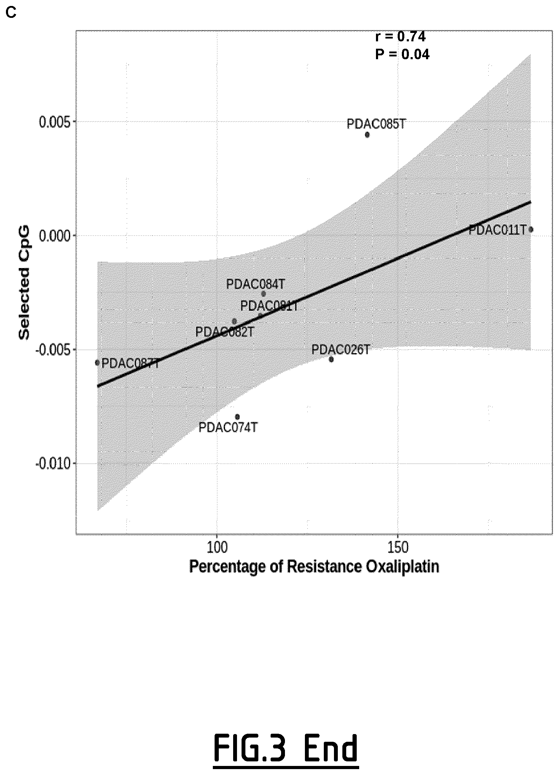

The refined signatures were evaluated *in vivo* on an independent cohort of 8 PDX randomly extracted from the validation cohort. All the signatures displayed a high chemosensitivity prediction capability (*see* *Figure 3*). Gemcitabine (r = -0.79; P = 0.01; Figure 3A), Irinotecan (r = -0.81; P = 0.01; Figure 3B), and Oxaliplatin (r = 0.74; P = 0.04; Figure 3C) signatures showed a strong predictive power, regarding to the chemosensitivity measured trough the cell lines.

## Claims

1. A method for determining whether a subject suffering from PDAC will respond favorably to at least one treatment, comprising:
(i) providing at least one set of CpG sites whose methylation level is associated to the response to said treatment,
(ii) determining the methylation level of the CpG sites of said set in a sample from said subject, and
(iii) deducing from the results of step (ii) whether the subject will respond favorably to said treatment.

2. The method according to claim 1, wherein said treatment comprises Gemcitabine, Irinotecan, Oxaliplatin, Fluorouracil (5-FU), Cisplatin, Abraxane and/or Taxol.

3. The method according to claim 1 or 2, wherein the sample originates from the pancreatic ductal adenocarcinoma of said subject.

4. The method according to any one of claims 1 to 3, wherein said sample is a DNA sample.

5. The method according to any one of claims 1 to 4, wherein step (i) comprises providing a set of CpG sites whose methylation level is associated to the response to Gemcitabine, a set of CpG sites whose methylation level is associated to the response to Irinotecan and/or a set of CpG sites whose methylation level is associated to the response to Oxaliplatin.

6. The method of claim 5, wherein the set of CpG sites whose methylation level is associated to the response to Gemcitabine comprises at least four CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7.

7. The method of claim 5, wherein the set of CpG sites whose methylation level is associated to the response to Irinotecan comprises at least four CpG sites selected from the group consisting of the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16.

8. The method of claim 5, wherein the set of CpG sites whose methylation level is associated to the response to Oxaliplatin comprises at least three CpG sites selected from the group consisting of the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20.

9. A kit suitable for determining whether a subject suffering from PDAC will respond favorably to a treatment, wherein said kit comprises:
(i) means for assessing the methylation level of a set of at most 60 CpG sites, wherein the CpG sites comprise:
- at least four CpG sites selected from the group consisting of the CpG located at position 61-62 of sequence SEQ ID NO: 1, the CpG located at position 61-62 of SEQ ID NO: 2, the CpG located at position 61-62 of sequence SEQ ID NO: 3, the CpG located at position 61-62 of sequence SEQ ID NO: 4, the CpG located at position 61-62 of sequence SEQ ID NO: 5, the CpG located at position 61-62 of sequence SEQ ID NO: 6 and the CpG located at position 61-62 of sequence SEQ ID NO: 7, and/or
- at least four CpG sites selected from the group consisting the CpG located at position 61-62 of sequence SEQ ID NO: 8, the CpG located at position 61-62 of SEQ ID NO: 9, the CpG located at position 61-62 of sequence SEQ ID NO: 10, the CpG located at position 61-62 of sequence SEQ ID NO: 11, the CpG located at position 61-62 of sequence SEQ ID NO: 12, the CpG located at position 61-62 of sequence SEQ ID NO: 13, the CpG located at position 61-62 of sequence SEQ ID NO: 14, the CpG located at position 61-62 of sequence SEQ ID NO: 15 and the CpG located at position 61-62 of SEQ ID NO: 16, and/or
- at least three CpG sites selected from the group consisting the CpG located at position 61-62 of sequence SEQ ID NO: 17, the CpG located at position 61-62 of sequence SEQ ID NO: 18, the CpG located at position 61-62 of sequence SEQ ID NO: 19 and the CpG located at position 61-62 of sequence SEQ ID NO: 20, and
(ii) optionally, means for extracting DNA from a sample.

10. The kit according to claim 9, wherein the means for assessing the methylation level of the CpG sites comprise:
- bisulfite,
- means for DNA amplification, and/or
- probes able to detect a methylated cytosine and/or an unmethylated cytosine at each CpG site of the set of CpG sites.

11. Use of the kit according to claim 9 or 10 for determining the methylation level of CpG sites in a sample.

12. The use according to claim 11, for determining whether a subject suffering from PDAC will respond favorably to a treatment.

13. A pharmaceutical composition for use in the treatment of PDAC in a subject, wherein said pharmaceutical composition comprises a treatment suitable for said subject, wherein said suitable treatment is selected by performing the method for determining whether a subject suffering from PDAC will respond favorably to at least one treatment according to any one of claims 1 to 8 and selecting a treatment to which the subject will respond favorably.
